# EUROPEAN PATENT APPLICATION

(11) **EP 3 375 371 A1**
(43) Date of publication of application: **19.09.2018**
(21) Application number: 17160532.2
(22) Date of filing: 13.03.2017
(51) Int. Cl.: A61B 5/11, A61B 5/00, A45D 26/00

(54) **A SYSTEM, APPARATUS AND METHOD OF ESTIMATING THE LOCATION AND/OR ORIENTATION OF A HANDHELD PERSONAL CARE DEVICE WITH RESPECT TO A USER**

(71) Applicant: Koninklijke Philips N.V., 5656 AE Eindhoven (NL)
(72) Inventor: Palero, Jonathan, 5656 AE Eindhoven (NL); Jurna, Martin, 5656 AE Eindhoven (NL); Visweswara, Ashoka Sathanur, 5656 AE Eindhoven (NL); Buil, Vincent, 5656 AE Eindhoven (NL)
(74) Representative: de Haan, Poul Erik

(57) **Abstract**

There is provided a method of estimating a location and/or orientation of a handheld personal care device with respect to a user of the handheld personal care device. The method includes obtaining measurements of a posture of a part of the body of the user as the user uses the handheld personal care device. The method also includes using the measurements to estimate the location and/or orientation of the handheld personal care device with respect to the user during use of the handheld personal care device based on a predetermined relationship between the location and/or orientation of the handheld personal care device and the posture of the part of the body of the user. A personal care system, a posture measurement device and a machine-readable medium are also disclosed.

## Description

### TECHNICAL FIELD OF THE INVENTION

The invention relates to a system and method for estimating a location and/or orientation of a personal care device with respect to a user.

### BACKGROUND TO THE INVENTION

Personal care devices are used to perform personal care activities on, for example, skin of a user. Such personal care activities include, but are not limited to, skin care activities (e.g. skin rejuvenation, skin stimulation and product application), hair care activities (e.g. hair shaving, epilating, hair trimming and hair drying) or massaging. While the user may know which part of his or her body is being treated by the personal care device, and may be able to tailor the application of the personal care device accordingly, the device itself may function in a suboptimal manner for some parts of the body compared to others.

Furthermore, some personal care systems include cameras which are capable of capturing images of large portions of a user or their body, including, for example, their face. Since some personal care activities may be considered to be private or intimate in nature, it may be undesirable to use a system in which the camera is capable of capturing large images, particularly if the system is capable of connecting to, and transmitting data to and from, a network, such as the internet.

Some personal care systems require numerous sensors, including a sensor located on or within the personal care device itself, to determine the position of the device relative to the user or to another device.

### SUMMARY OF THE INVENTION

In order to operate in an optimal manner, it can be beneficial to identify an approximate location and/or orientation of a personal care device relative to the body of a user of the device, for example by identifying a part of the body to which a personal care activity is being performed, and the orientation in which the personal care device is being held. Existing means for determining a location of a device can be ineffective. Furthermore, some existing means can require images to be acquired, or data to be obtained from multiple sensors. Therefore, there is a desire for a more effective means for estimating the location and/or orientation of a personal care device with respect to a user of the device.

The inventors have realised that a location, an orientation, or both a location and an orientation of a device can be estimated if a posture of a part of the user's body is known.

According to a first aspect of the invention, there is provided a method of estimating a location and/or orientation of a handheld personal care device with respect to a user of the handheld personal care device, the method comprising: obtaining measurements of a posture of a part of the body of the user as the user uses the handheld personal care device; and using the measurements to estimate the location and/or orientation of the handheld personal care device with respect to the user during use of the handheld personal care device based on a predetermined relationship between the location and/or orientation of the handheld personal care device and the posture of the part of the body of the user.

In this way, an estimate of the orientation and/or the location can be made without the need for a camera. Thus, privacy of the user is ensured. Moreover, a mere estimate of the location of the personal care device may be sufficient to enable the device to operate in a safer or more optimal manner.

Obtaining measurements may comprise measuring a parameter of the part of the body, the parameter being indicative of an orientation of the part of the body.

In some embodiments, the measurements of the posture of the part of the body of the user are obtained using a posture measurement device attached to the part of the body.

Estimating the location and/or orientation may, in some embodiments, comprise consulting a lookup table or database comprising the predetermined relationship between the posture of the part of the body of the user and the location and/or orientation of the handheld personal care device. Data in the look-up table may be entered manually, for example by a user, or by the device manufacturer. In some cases, the device may be used in a calibration mode, whereby the user moves the device as if in use, and movements of a body part may be measured as the device is moved.

The method may further comprise adjusting a parameter of the handheld personal care device based on the estimation of the location and/or orientation of the handheld personal care device with respect to the user.

In some embodiments, obtaining measurements comprises obtaining measurements of the posture of a particular part of the body of the user as the user uses the handheld personal care device on the particular part of the body of the user.

According to a second aspect of the invention, there is provided an apparatus for estimating a location and/or orientation of a handheld personal care device with respect to a user, the apparatus comprising processing apparatus configured to: obtain measurements of a posture of a part of the body of the user as the user uses the handheld personal care device; and estimate the location and/or orientation of the handheld personal care device with respect to the user during use of the handheld personal care device based on a predetermined relationship between the location and/or orientation of the handheld personal care device and the posture of the part of the body of the user.

The predetermined relationship may define a particular location and/or orientation of the handheld personal care device for each of a plurality of particular postures of a particular part of the body of the user.

According to a third aspect of the invention, there is provided a personal care system comprising: a handheld personal care device; a posture measurement device for measuring a posture of a part of the body of a user; and an apparatus as described above in accordance with the second aspect of the invention.

In some embodiments, the posture measurement device may comprise a sensor for measuring a parameter indicative of the posture of the part of the body; and a controller. The posture measurement device may comprise communication circuitry for enabling communication between the posture measurement device and one or more of the handheld personal care device and the processing apparatus.

In some embodiments, the posture measurement device may comprise at least one of: electromyography (EMG) instrumentation; body joint flex sensing instrumentation; an inertial measurement unit (IMU); an imaging device; and a range-finding device.

The posture measurement device may, in some embodiments, comprise a mount for mounting the posture measurement device to the part of the body of the user.

In some embodiments, the handheld personal care device may comprise a further sensor for measuring an orientation and/or change in location of the handheld personal care device relative to the user. The processing apparatus may be configured to estimate the location and/or orientation of the handheld personal care device with respect to the user during use of the handheld personal care device based on a measurement from the further sensor and on the predetermined relationship.

According to a fourth aspect of the invention, there is provided a posture measurement device comprising: a sensor configured to measure a parameter indicative of a posture of a part of a body, the posture corresponding, by a predetermined relationship, to a location and/or orientation of a handheld personal care device with respect to the body; and communication circuitry configured to transmit the measured parameter to processing apparatus for estimating the location and/or orientation of the handheld personal care device based on the predetermined relationship.

According to a fifth aspect of the invention, there is provided a machine-readable medium comprising instructions which, when executed by a processor, cause the processor to: obtain measurements of a posture of a part of the body of a user of a handheld personal care device as the user uses the handheld personal care device; and use the measurements to estimate a location and/or orientation of the handheld personal care device with respect to the user during use of the handheld personal care device based on a predetermined relationship between the location and/or orientation of the handheld personal care device and the posture of the part of the body of the user.

The machine-readable medium may comprise instructions which, when executed by a processor, cause the processor to: adjust a parameter of the handheld personal care device based on the estimation of the location and/or orientation of the handheld personal care device with respect to the user.

Other advantageous features will become apparent from the following description of the preferred embodiments.

### BRIEF DESCRIPTION OF THE DRAWINGS

For a better understanding of the invention, and to show more clearly how it may be carried into effect, reference will now be made, by way of example only, to the accompanying drawings, in which:
Figure 1 is a schematic illustration of an example of a personal care system according to embodiments of the invention;
Figure 2 is an illustration of a further example of a personal care system according to embodiments of the invention;
Figure 3 is an illustration of a further example of a personal care system according to embodiments of the invention;
Figure 4 is an illustration of an example of a posture measurement device according to embodiments of the invention;
Figure 5 is a schematic illustration of a further example of a posture measurement device according to embodiments of the invention;
Figure 6 is a schematic illustration of an example of a personal care device according to embodiments of the invention;
Figure 7 is a flowchart of an example of a method of estimating the location and/or orientation of a handheld personal care device according to embodiments of the invention;
Figure 8 is a chart showing an example of a relationship between a measured orientation of a body part and a position of a handheld personal care device;
Figure 9 is a flowchart of a further example of a method of estimating the location and/or orientation of a handheld personal care device according to embodiments of the invention; and
Figure 10 is a schematic view of an example of a machine-readable medium with a processor.

### DETAILED DESCRIPTION OF THE PREFERRED EMBODIMENTS

It can be desirable to determine a location and/or orientation of a personal care device during use. The location and/or orientation information may be used to determine information regarding the operation of the device. In some cases, the information may be used to set or adjust operating parameters of the device. When using a handheld personal care device, a user may move a part of his or her body in a particular position, location or orientation to enable him or her to perform a personal care activity to that, or some other, part of their body. The inventors of the present invention have realised that an estimation of the location and/or the orientation of a personal care device with respect to the body can be made based on knowledge of a posture of a part of the body of the user.

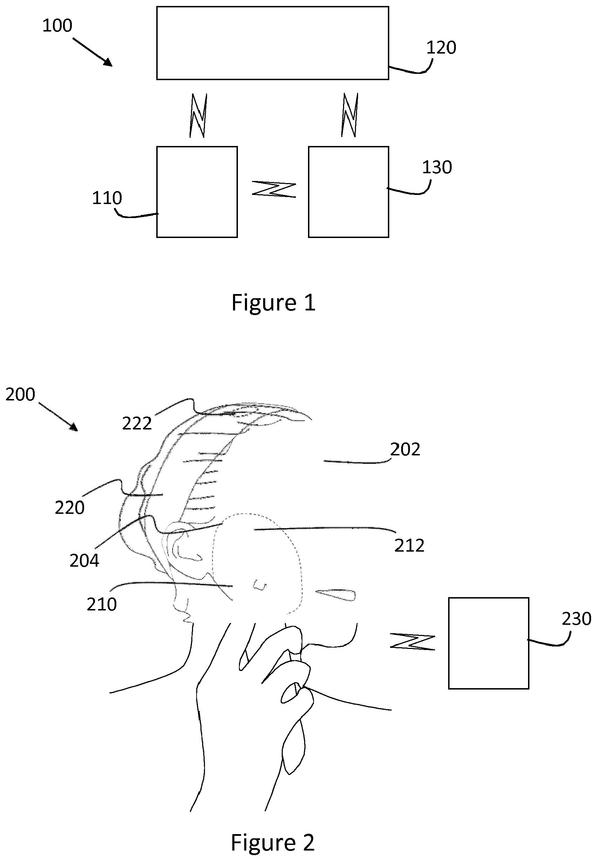

Aspects of the invention relate to a personal care system. Figure 1 shows, schematically, an example of a personal care system 100. The personal care system 100 comprises a personal care device 110 (e.g. a handheld personal care device), a posture measurement device 120 and processing apparatus 130. The personal care device 110, the posture measurement device 120 and the processing apparatus 130 may communicate with one another and, in some embodiments, with other devices, via a wired or wireless connection. For example, communication may be enabled via a Bluetooth connection or a wireless local area network (WLAN) connection, such as Wi-Fi.

The personal care device 110 may comprise any device capable of being used to perform a personal care activity on a user. Thus, the personal care device 110 may, for example, comprise a hair removal device, such as an epilator or an intense pulsed light (IPL) device, a hair care device, such as a shaver, clipper or a hair trimmer, a skin health analysis device, an electric massager, a phototherapy device, a skin cleansing device, a skin rejuvenation device, a skin sensing device or a pain relief device. Other personal care devices are also envisaged.

The posture measurement device 120 may comprise any device capable of measuring a posture of a part of the body of a user. As discussed in greater detail below, the posture measurement device 120 may be attached to a body part of the user. In some embodiments, the posture measurement device 120 may include one or more sensors for measuring a parameter of a body part indicative of the posture of the body part. The posture measurement device 120 may communicate any measured data (such as information relating to the posture of the body part) to the processing apparatus 130 for interpretation and/or analysis.

In some embodiments, the processing apparatus 130 maybe located remote from the personal care device 110 and/or the posture measurement device 120. In other embodiments, the processing apparatus 130 maybe located within the personal care device 110. In some embodiments, the processing apparatus 130 may form part of a computing device, such as a desktop computer, a laptop computer, a tablet computer, a smart phone, a smart watch, or the like. As discussed below, the personal care device 110 and the posture measurement device 120 may each have their own processing apparatus or control circuitry.

The processing apparatus 130 is configured to estimate the location and/or orientation of the handheld personal care device 110 with respect to the user during use of the handheld personal care device based on a predetermined relationship between the location and/or orientation of the handheld personal care device and the posture of the part of the body of the user. In some embodiments, the predetermined relationship between the location and/or orientation of the handheld personal care device 110 and the posture of the part of the body of the user may be stored in the form of a look-up table or database. For example, a user might move a particular body part into a particular posture when performing a particular personal care activity on a particular part of his or her body. Thus, a particular posture of a part of the user's body might be associated with a particular orientation or location of, or combination of orientation and location of, the personal care device 110.

The expression "processing apparatus" is used herein to refer to an entity or system for processing, for example, those that process in response to a signal or data and/or those that process autonomously. Processing apparatus should be understood to encompass microprocessors, microcontrollers, programmable digital signal processors, integrated circuits, computer software, computer hardware, electrical circuits, application specific integrated circuits, programmable logic devices, programmable gate arrays, programmable array logic, personal computers, chips, and any other combination of discrete analogue, digital, or programmable components, or other devices capable of providing processing functions.

Aspects of the invention relate to an apparatus for estimating the location and/or orientation of a handheld personal care device with respect to a user. The apparatus comprises processing apparatus (such as processing apparatus 130), which is configured to obtain measurements of a posture of a part of the body of the user as the user uses the handheld personal care device; and estimate the location and/or orientation of the handheld personal care device with respect to the user during use of the handheld personal care device based on a predetermined relationship between the location and/or orientation of the handheld personal care device and the posture of the part of the body of the user.

The predetermined relationship may define a location and/or orientation of the handheld personal care device for each of a plurality of particular postures of a particular part of the body of the user. The predetermined relationship may form an entry in a database or look-up table as described below.

According to some aspects, the invention relates to a personal care system comprising a handheld personal care device (such as the device 110); a posture measurement device (such as the device 120) for measuring the posture of a part of the body of a user; and the apparatus 150 described above. The apparatus 150 may comprise a computing device, such as smartphone, a smart watch, a tablet computer or a laptop computer. In other embodiments, the apparatus 150 may comprise any other device having processing apparatus configured to operate in the manner described above.

Figure 2 shows an illustration of an example of a personal care system 200 being used by a user 202. The personal care system 200 includes a personal care device 210, a posture measurement device 220 and processing apparatus 230. In Figure 2, the user 202 is holding the handheld personal care device 210 which, in this embodiment, is a skin cleansing device. The skin cleansing device 210 includes a personal care element 212 which, in this example is a brush element. The brush element 212 includes a plurality of bristles, and the brush element is caused to rotate either clockwise or anti-clockwise relative to the skin cleansing device 210. The user 202 holds the skin cleansing device 210 such that the bristles of the brush element 212 brush against the user's skin. A dashed ring 204 indicates an area of the skin of the user 202 being treated while the skin cleansing device 210 is held as shown in Figure 2.

While performing the personal care activity using the personal care device (e.g. the skin cleansing device 210), the user wears the posture measurement device 220. In the embodiment shown in Figure 2, the posture measurement device 220 comprises a headband worn around the head of the user 202. The headband 220 comprises a measurement element 222 which includes a sensor (not shown in Figure 2). In this embodiment, the sensor is configured to measure the posture of the user's head. In other embodiments, the sensor of the measurement element 222 may be attached to, and configured to measure the posture of, a different part of the user's body. In some embodiments, the sensor of the measurement element 222 may be configured to measure a parameter of a part of the body of the user (e.g. a parameter of the part of the user's body to which the posture measurement device 220 is attached), the parameter being indicative of a posture of the body part.

The measurement element 222 measures a posture of a body part of the user 202 in a manner as described in detail below. The measured posture is, in some embodiments, then transmitted to the processing apparatus 230 either via a wire or cable, or wirelessly. In this embodiment, the processing apparatus 230 is located remote from the personal care device 210 and the posture measurement device 220. Upon receipt of a signal (e.g. a signal indicating the nature of the measured posture of the body part) from the measurement element 222, the processing apparatus 230 may interrogate or consult a database or look-up table for a location or position of the skin cleansing device 210 corresponding to the measured posture of the part of the body (i.e. the head in this embodiment). The database or look-up table may additionally or alternatively include information indicative of an orientation of the skin cleansing device 210 corresponding to the measured posture. In the example shown in Figure 2, if the measurement element 222 detects that the user's head is tilted to the left (from the user's perspective), then the database or look-up table may indicate that the skin cleansing device is being used on the right cheek.

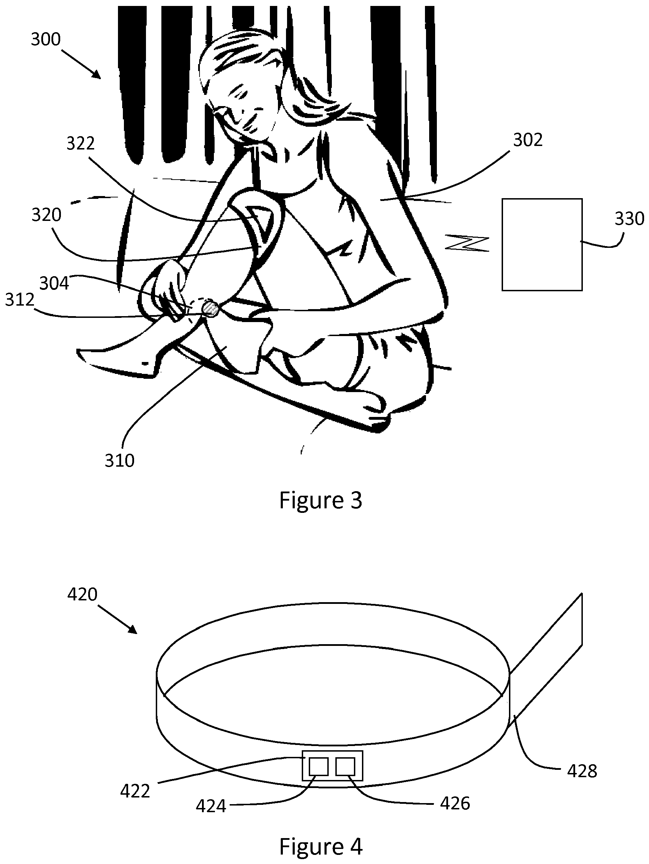

Figure 3 shows an illustration of a further example of a personal care system 300 being used by a user 302. The personal care system 300 includes a personal care device 310, a posture measurement device 320 and processing apparatus 330. In Figure 3, the user 302 is holding the handheld personal care device 310 which, in this embodiment, is an intense pulsed light (IPL) device used for removing hair from the user's body. The IPL device 310 includes a personal care element 312 which, in this example, is a light emitting element. The light emitting element 312 is configured to emit pulses of light towards skin of the user 302 to reduce hair growth. The user 302 holds the IPL device 310 such that the light emitting element 312 is against or near to his or her skin in the region to be treated. A dashed ring 304 indicates an area of the skin of the user 302 being treated while the IPL device 310 is held as shown in Figure 3.

While performing the personal care activity shown in Figure 3 using the personal care device (e.g. the IPL device 310), the user wears a posture measurement device 320. In the embodiment shown in Figure 3, the posture measurement device 320 comprises a strap worn around the knee of the user 302. As with the posture measurement device 220 shown in Figure 2, the strap 320 of the embodiment of Figure 3 comprises a measurement element 322 which includes a sensor (not shown in Figure 3). In this embodiment, the sensor is configured to measure the posture of the user's knee and/or a portion of the user's lower leg (i.e. below the knee). In other embodiments, the sensor of the measurement element 322 may be attached to, and configured to measure the posture or a parameter of, a different part of the user's body (e.g. a foot or an ankle).

The measured posture may be transmitted to the processing apparatus 330 either via a wire or cable, or wirelessly. In this embodiment, the processing apparatus 330 is located remote from the personal care device 310 and the posture measurement device 320. As in the embodiment shown in Figure 2, upon receipt of a signal (e.g. a signal indicating the nature of the measured posture of the body part) from the measurement element 322, the processing apparatus 330 may interrogate or consult a database or look-up table for a location or position of the IPL device 310 corresponding to the measured posture of the part of the body (i.e. the user's leg in this embodiment). The database or look-up table may additionally or alternatively include information indicative of an orientation of the IPL device 210 corresponding to the measured posture. In the example shown in Figure 3, the tilting of the user's leg to the user's left or right and the amount of bending at the knee might be indicative of a particular area of the leg at which the IPL device is being used. For example, if the user bend's their leg and tilts their knee to the user's left, the database or look-up table may indicate that the user is treating the right-hand side of their lower leg.

The posture measurement device 120, 220, 320 may be sized and shaped according to the body part to be monitored (i.e. the body part whose posture is to be measured). In some embodiments, the posture measurement device may be configured to measure a posture of a part of a user's body while it is positioned remote from the body part. In other embodiments, such as the embodiments discussed above with reference to Figures 2 and 3, the posture measurement device may be positioned on, or attached to a body part whose posture is to be measured. The posture measurement device may, in some examples, be attached to a part of the user's body, but maybe configured to measure the posture of a different part of the user's body. For example, the posture measurement device may be attached to a user's leg, and configured to measure the posture of a user's foot, using muscle movements detected in the leg.

A further example of a posture measurement device 420 is shown in Figure 4. The posture measurement device 420 comprises a measurement element 422, which may include one or more sensors 424 (only one sensor 424 is shown in Figure 4). The sensor 424 (or at least one sensor where multiple sensors are provided) is configured to measure a posture of a body part. The measurement element 422 may include a transmitter 426 for transmitting measurements acquired by the sensor 424 to processing apparatus, such as processing apparatus 130, 230, 330, for analysis. The transmission of data by the transmitter 426 may be made via a wired or wireless connection. In some embodiments, the posture measurement device 420 or the measurement element 422 comprises a receiver (not shown) for receiving signals, such as instruction signals, from a computing device, for example. Such instruction signals may include instructions for the measurement element 422 to take a measurement of a posture of the body part. The posture measurement device may include control circuitry or processing circuitry for processing signals received by the receiver, and/or for processing, or pre-processing, data acquired by the sensor 424. The processing circuity may serve as control circuitry for controlling the measurement element 422.

In the embodiment shown in Figure 4, the posture measurement device 420 is a strap capable of being attached to a part of a user's body, such as a head, a neck, an arm, a wrist, a hand, a waist, a leg, an ankle or a foot. The posture measurement device 420 includes an attachment element 428 to enable to the posture measurement device to be attached to a body part. The attachment element 428 may enable the length of the posture measurement device 420 to be adjusted as desired. The attachment element 428 may comprise, for example, a buckle, a clip, or a hook and loop attachment means.

In order for the posture measurement device 420 to accurately measure a posture of a body part, it may be desirable, in some embodiments, for the measurement element 422, to be mounted or attached to the body part in a particular orientation. For example, in order to accurately measure the posture of the user's head in Figure 2, the headband 220 should be positioned such that the measurement element 222 is central with respect to the user's face, and at the highest point of the headband. To aid correct positioning, the posture measurement device 420 may, in some embodiments, include an indicator (not shown), such as a position indicator, an orientation indicator, or text or an image to indicate to a user how the posture measurement device should be positioned for optimum accuracy.

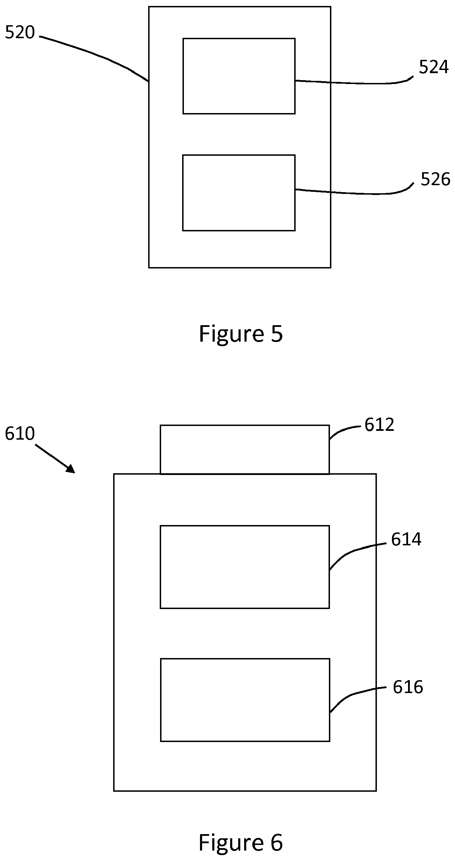

Figure 5 shows, schematically, a posture measurement device 520, similar to the devices 120, 220, 320 and 420 discussed above. The posture measurement device 520 comprises a sensor 522 configured to measure a parameter indicative of a posture of the part of a body, the posture corresponding, by a predetermined relationship, to a location and/or orientation of a handheld personal care device. The posture measurement device 520 further comprises communication circuitry 526 configured to transmit the measured parameter to processing apparatus for estimating the location and/or orientation of the handheld personal care device based on the predetermined relationship.

In some embodiments, the posture measurement device 520 may comprise a controller, or control circuitry, for example to control the sensor 522. In some embodiments, the communication circuitry 526 may be for enabling communication between the posture measurement device 520 and one or more of the handheld personal care device (such as the devices 110, 210, 310) and the processing apparatus (such as the processing apparatus 130, 230, 330).

The posture measurement device 120, 220, 320, 420, 520 (or the measurement element or sensor of the posture measurement device) may comprise at least one of electromyography (EMG) instrumentation; body joint flex sensing instrumentation; an inertial measurement unit (IMU); an imaging device; and a range-finding device. EMG instrumentation may be used to evaluate electrical activity produced by skeletal muscles. By analysing the electrical activity, a measure of the posture of the muscle (and, therefore, the associated body part) can be determined. Joint flex sensing instrumentation may be used to measure the amount of flexing in a joint of a user as the user moves a body part. The amount of flexing may be used as an indicator of the posture of the body part. An inertial measurement unit may be used to measure orientation of a body part. By analysing the orientation, a measure of the posture of the body part may be determined. An imaging device may be used to image at least part of the user's body so that a skeleton image (e.g. a 'stick-man' or line representation) of the user or a body part of the user can be generated. From the generated representation, a posture of a body part may be determined. A range-finding device may be used to determine a posture of a body part. For example, a proximity-sensing device may be used to determine a distance between two locations of a user's body. In one example, a wearable device located on a body part of the user may communicate with the handheld personal care device and/or a wearable device located on another body part, for example using Bluetooth ranging, to determine a posture.

The posture measurement device 520 may comprise a mount, such as the attachment element discussed above, for mounting the posture measurement device to a part of the body of the user.

As noted above, the personal care device 110, 210, 310 may comprise any device capable of being used to perform a personal care activity, and examples are discussed above. The personal care device includes a personal care element (such as the brush element 212 or the light emitting element 312 discussed above). In general, the personal care element is a portion of the personal care device 110, 210, 310 that performs the personal care activity, and it will be appreciated that the nature of the personal care element will depend on the nature of the particular personal care device.

A further example of a handheld personal care device 610 constructed in accordance with some embodiments of the invention is shown in Figure 6. The handheld personal care device 610 includes a personal care element 612. The personal care device 610 also comprises a further sensor 614 for measuring an orientation and/or change in location of the handheld personal care device relative to the user. The further sensor 614 may, for example, comprise an accelerometer, a gyroscope and/or an IMU. Processing apparatus (such as the processing apparatus 130, 230, 330) may be configured to estimate the location and/or orientation of the handheld personal care device 610 with respect to the user during use of the handheld personal care device based on a measurement from the further sensor 614 and on the predetermined relationship. Thus, using measurements from both the posture measurement device and the further sensor 614 in the handheld personal care device 610, a more accurate measure of the posture and/or the orientation and/or the location of the user's body part may be determined.

The handheld personal care device 610 may, in some embodiments, comprise communication circuitry 616 for enabling communication between the handheld personal care device and at least one of the posture measurement device and the processing apparatus.

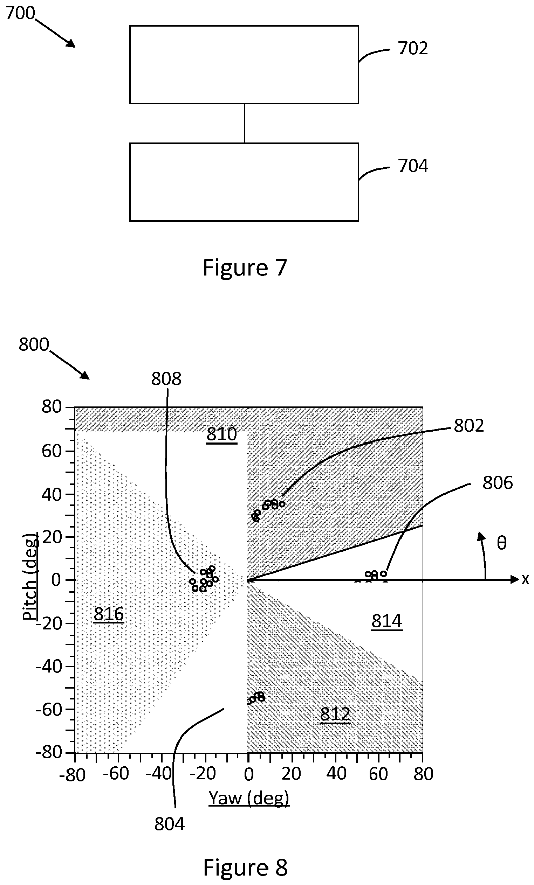

A further aspect of the invention relates to a method of estimating a location and/or orientation of a handheld personal care device with respect to a user of the handheld personal care device. Figure 7 is a flowchart of an example of such a method 700. The method 700 comprises, at step 702, obtaining measurements of a posture of a part of the body of the user as the user uses the handheld personal care device. As noted above, such measurements may be obtained using a posture measurement device, such as the devices 120, 220, 320, 420, 520. The method 700 comprises, at step 704, using the measurements to estimate the location and/or orientation of the handheld personal care device with respect to the user during use of the handheld personal care device based on a predetermined relationship between the location and/or orientation of the handheld personal care device and the posture of the part of the body of the user.

A particular posture of a body part may be indicative that the user is performing a particular personal care activity, or operating the handheld personal care device on a particular part of his or her body. For example, a person using a skin cleansing device to perform a skin cleansing treatment to the skin on his or her face may move his or her head into a particular position and/or orientation when using the device on a particular region of his or her face. Referring to the example shown in Figure 2, while the user 202 is using the skin cleansing device 210 on the right cheek, he or she tends to tilt the head upwards and to the left. Such a position may allow the user to view his or her reflection in a mirror. The posture measurement device 220 worn on the user's head may detect that the head has moved in a particular manner (e.g. upwards and to the left), and may send that information to the processing apparatus 230 for analysis. The processing apparatus 230 may refer to a database or look-up table, for example, to find a predetermined relationship between that particular head movement (i.e. posture) and an associated location and/or orientation of the handheld personal care device. For example, a look-up table may include an entry indicating that a movement of the head up and to the left corresponds to a user performing a personal care activity to the right cheek. As such, the processing apparatus 230 may estimate that the handheld personal care device is located near to the right cheek of the user, and that the handheld personal care device may be oriented approximately vertically (as that tends to be how such a device is held when treating the cheeks).

In some embodiments, the processing apparatus may itself be able to determine an approximate location of the posture measurement device (e.g. on a head, on an arm, or on a leg), so that an accurate determination (e.g. left cheek, forehead, left forearm, right inner thigh) can be made. In some embodiments, a user may manually input details of the part of a body to be treated, and/or the type of personal care device being used to perform the personal care activity.

Referring again now to the example shown in Figure 3, the user 302 is sitting down, using the IPL device 310 on an outer side (i.e. the left side) of the left leg. While using the device 310 in this manner, the user 302 tends to raise the left knee upwards. The knee may also be tilted towards the body of the user. Such a position may allow the user to reach the leg easily. The posture measurement device 320 worn on the user's knee may detect that the knee has moved in a particular manner (e.g. bent and moved upwards and towards the user's body), and may send that information to the processing apparatus 330 for analysis. The processing apparatus 330 may determine that the measured posture corresponds (according to a predetermined relationship) to a personal care activity being performed on the lower left leg of the user.

In some embodiments, the obtaining of measurements of the posture of the part of the user's body (step 702 of the method 700) comprises measuring a parameter of the part of the body, the parameter being indicative of an orientation of the part of the body. In other words, rather than using a sensor in the posture measurement device to determine, for example, an orientation of the part of the body, a parameter of the part of the body may be measured which can be used to determine the orientation of the part of the body. For example, such a parameter may include an amount of light reflected from a particular part of the body. In other examples, a shadow cast by a part of the user's body may be observer and/or imaged using an imaging device, such as a camera or a light sensitive panel. Alternatively, the acceleration of a moving part of the body may be measured using an IMU, and the acceleration may be used as a parameter from which to determine the orientation of the part of the body.

As discussed above, the estimation of the location and/or orientation of the handheld personal care device may comprise consulting a lookup table or database containing a predetermined relationship between the measurements of the posture of the part of the body of the user and the location and/or orientation of the handheld personal care device. The look-up table or database may be stored in a storage medium in a computing device associated with or remote from the processing apparatus, in the posture measurement device, or in the handheld personal care device. Data in the look-up table may be entered manually, for example by a user, or by the device manufacturer. Such data may be acquired by testing, for example. In some cases, data for the look-up table may be obtained by the personal care system itself. For example, the device may be used in a calibration mode, whereby the user moves the device as if in use, and corresponding movements of the part of the body may be measured as the device is moved.

Figure 8 is a graph 800 showing an example of a predetermined relationship between the location and/or orientation of a handheld personal care device and a posture of a part of the body of the user. The data in the graph of Figure 8 relates to the example shown in Figure 2, where a user 202 performs a personal care activity to the face using a skin cleansing device 210. The data is based on an orientation of the head of the user, as measured using the measurement element 222 of the headband 220, and is defined in terms of two parameters: yaw (i.e. left and right rotation of the head about a vertical axis) and pitch (i.e. up and down rotation of the head). In some embodiments, data may additionally or alternatively be defined in terms of a third parameter, e.g. roll (i.e. left and right tilting of the head). The graph of Figure 8 is a plot of the pitch rotation and yaw rotation (relative to an initial position in which the user's head faces forwards) of the head as the skin cleansing device is used in different locations on the user's face. The data assumes that the skin cleansing device is operated in the user's right hand. Thus, for a left-handed user, the data may be reversed.

From the graph 800, it is clear that use of the skin cleansing device in a particular region of the user's face corresponds to an orientation of the head in a particular way. Specifically, data points 802 correspond to measurements taken while the user tilts the head downwards to treat the forehead, data points 804 correspond to measurements taken while the user tilts the head upwards to treat the neck, data points 806 correspond to measurements taken while the user rotates the head to the left to treat the right cheek, and data points 808 correspond to measurements taken while the user rotates the head to the right to treat the left cheek. Thus, based on these measurements, a general rule can be established and entered into a look-up table. For example, if a measurement of a posture of the user's head falls within a shaded range 810 indicated in the graph, then it can be assumed that the personal care device is being used on the user's forehead; if a measurement of a posture of the user's head falls within a shaded range 812 indicated in the graph, then it can be assumed that the personal care device is being used on the user's neck; if a measurement of a posture of the user's head falls within a shaded range 814 indicated in the graph, then it can be assumed that the personal care device is being used on the user's right cheek; and if a measurement of a posture of the user's head falls within a shaded range 816 indicated in the graph, then it can be assumed that the personal care device is being used on the user's left cheek.

The example shown in the graph 800 of Figure 8 may be entered into a look-up table or database in terms of a polar angle (i.e. an angle *θ* of rotation from axis *x* in Figure 8), as follows:

| Polar angle, *θ*° | Location of personal care application |
|---|---|
| *θ*≤14° and *θ*>334° | Right cheek |
| 14°< *θ*≤144° | Forehead |
| 144°< *θ*≤228° | Left cheek |
| 228°< *θ*≤334° | Neck |

In this example, *θ* = arctan2(pitch, yaw).

In some embodiments, smaller ranges may be used, such that a more accurate location of application may be determined.

Once the location of the handheld personal care device has been estimated, the information may be used for various purposes. In some embodiments, the method 700 may further comprise storing at least one of the measured posture (or a parameter indicative of the posture), the estimated location and the estimated orientation of the personal care device in a storage medium, such as a memory. By storing the acquired data, the data may be used at a later time. For example, the stored data may be used to generate a log or a record of the locations on the user's body where the personal care activity has been performed. Such information may be used to provide an indication to the user of parts of his or her body that have not yet been treated, or portions of his or her body to which the personal care activity has been performed too many times. In this way, feedback may be provided in real time and/or subsequently to the user regarding a quality of the user's performance of the personal care activity.

In some embodiments, the method may further comprise transmitting at least one of the measured posture (or a parameter indicative of the posture), the estimated location and the estimated orientation of the personal care device to a computing device. In other words, at least some of the acquired data may be transmitted, for example via a wired or wireless connection, to a connected or remote computing device. The transmitted data may be used for further analysis, for example by the user or by a third party. The data may, in some embodiments, be transmitted to a medical professional, particularly if the personal care activity involves monitoring or measuring a parameter of the user's skin. Additionally or alternatively, the transmitted data may be delivered to a manufacturer of the personal care device to enable the manufacturer to determine the extent to which the personal care device is used on different parts of the body and/or to enable the manufacturer to track the usage of the device so that the user may be informed when the personal care element is due to be replaced, for example.

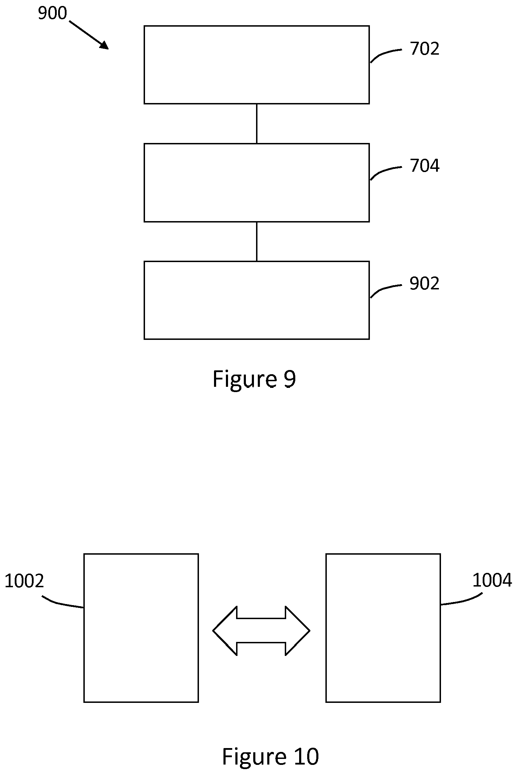

Figure 9 is a flowchart of an example of a method 900 for estimating the location and/or orientation of a handheld personal care device with respect to a user. The method 900 includes the steps 702 and 704 discussed above with reference to Figure 7. The method 900 further comprises, at step 902, adjusting a parameter of the handheld personal care device based on the estimation of the location and/or orientation of the handheld personal care device with respect to the user. In some embodiments, the parameter may be an operating parameter (e.g. a parameter that affects the operation of the device). The personal care element 212, 312, 612 of the personal care device 110, 210, 310, 610 may be caused to operate in accordance with one or more operating parameters which may depend on the nature of the personal care element, and/or on the nature of the user (e.g. male or female). However, the personal care element may be caused to operate in accordance with one or more parameters which can be adjusted or tailored depending on the body part on which the personal care activity is being performed. For example, in an embodiment in which the personal care element comprises a hair removal element, such as a photo-epilation element or a light emitting element of an IPL device, an intensity and/or a wavelength of radiation to be emitted by the personal care element may depend upon the type of hair to be treated and, therefore, on the body part from which the hair is growing. The treatment of relatively thick hair may require a greater intensity than relatively thin hair, for example. Similarly, a relatively greater intensity of light may be applied to a hair growing deeper in the skin of the body of the user than to a hair which is growing shallower in the skin. Thus, the processing apparatus may adjust, or instruct the personal care device to adjust, one or more parameters of the personal care element based on the estimation of the location and/or orientation of the personal care device made in step 704.

In some embodiments, one or more optimum settings or parameters maybe stored (for example in a storage unit of the personal care device) which correspond to the various body parts, or to particular locations and/or orientations of the personal care device. Thus, when a particular location is estimated in step 704, the processing apparatus may automatically adjust the operating parameters of the personal care element to suit the body part being treated, such that the personal care activity is performed in an optimised manner. In other embodiments, a user may manually adjust one or more parameters based on the estimated body part.

A further aspect of the invention relates to a machine-readable medium (e.g. a computer-readable medium) which may comprise instructions which, when executed by a processor or processing apparatus, cause the processor or processing apparatus to perform the methods disclosed herein. Figure 10 shows an example of a machine readable medium 1002 with a processor 1004, which may be similar to the processing apparatus 130, 230 or 330. The machine-readable medium 1002 comprises instructions which, when executed by the processor 1004, cause the processor to obtain measurements of a posture of a part of the body of a user as the user uses a handheld personal care device; and use the measurements to estimate a location and/or orientation of the handheld personal care device with respect to the user during use of the handheld personal care device based on a predetermined relationship between the location and/or orientation of the handheld personal care device and the posture of the part of the body of the user.

In some embodiments, the machine-readable medium 1002 may comprise instructions which, when executed by the processor 1004, cause the processor to adjust a parameter of the handheld personal care device based on the estimation of the location and/or orientation of the handheld personal care device with respect to the user.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. Any reference signs in the claims should not be construed as limiting the scope.

## Claims

1. A method of estimating a location and/or orientation of a handheld personal care device with respect to a user of the handheld personal care device, the method comprising:
obtaining measurements of a posture of a part of the body of the user as the user uses the handheld personal care device; and
using the measurements to estimate the location and/or orientation of the handheld personal care device with respect to the user during use of the handheld personal care device based on a predetermined relationship between the location and/or orientation of the handheld personal care device and the posture of the part of the body of the user.

2. A method according to claim 1, wherein obtaining measurements comprises: measuring a parameter of the part of the body, the parameter being indicative of an orientation of the part of the body.

3. A method according to claim 1 or claim 2, wherein the measurements of the posture of the part of the body of the user are obtained using a posture measurement device attached to the part of the body.

4. A method according to any one of the preceding claims, wherein said estimating comprises:
consulting a lookup table or database comprising the predetermined relationship between the posture of the part of the body of the user and the location and/or orientation of the handheld personal care device.

5. A method according to any one of the preceding claims, further comprising: adjusting a parameter of the handheld personal care device based on the estimation of the location and/or orientation of the handheld personal care device with respect to the user.

6. A method according to any one of the preceding claims, wherein obtaining measurements comprises:
obtaining measurements of the posture of a particular part of the body of the user as the user uses the handheld personal care device on the particular part of the body of the user.

7. An apparatus for estimating a location and/or orientation of a handheld personal care device with respect to a user, the apparatus comprising:
processing apparatus configured to:
obtain measurements of a posture of a part of the body of the user as the user uses the handheld personal care device; and
estimate the location and/or orientation of the handheld personal care device with respect to the user during use of the handheld personal care device based on a predetermined relationship between the location and/or orientation of the handheld personal care device and the posture of the part of the body of the user.

8. An apparatus according to claim 7, wherein the predetermined relationship defines a particular location and/or orientation of the handheld personal care device for each of a plurality of particular postures of a particular part of the body of the user.

9. A personal care system comprising:
a handheld personal care device;
a posture measurement device for measuring a posture of a part of the body of a user; and
an apparatus according to claim 7 or claim 8.

10. A personal care system according to claim 9, wherein the posture measurement device comprises:
a sensor for measuring a parameter indicative of the posture of the part of the body; and
a controller.

11. A personal care system according to claim 9 or claim 10, wherein the posture measurement device comprises:
communication circuitry for enabling communication between the posture measurement device and one or more of the handheld personal care device and the processing apparatus.

12. A personal care system according to any one of the claims 9 to 11, wherein the posture measurement device comprises at least one of: electromyography (EMG) instrumentation; body joint flex sensing instrumentation; an inertial measurement unit (IMU); an imaging device; and a range-finding device.

13. A personal system according to any one of the claims 9 to 12, wherein the handheld personal care device comprises:
a further sensor for measuring an orientation and/or change in location of the handheld personal care device relative to the user;
wherein the processing apparatus is configured to estimate the location and/or orientation of the handheld personal care device with respect to the user during use of the handheld personal care device based on a measurement from the further sensor and on the predetermined relationship.

14. A posture measurement device comprising:
a sensor configured to measure a parameter indicative of a posture of a part of a body, the posture corresponding, by a predetermined relationship, to a location and/or orientation of a handheld personal care device with respect to the body; and
communication circuitry configured to transmit the measured parameter to processing apparatus for estimating the location and/or orientation of the handheld personal care device based on the predetermined relationship.

15. A machine-readable medium comprising instructions which, when executed by a processor, cause the processor to:
obtain measurements of a posture of a part of the body of a user of a handheld personal care device as the user uses the handheld personal care device; and
use the measurements to estimate a location and/or orientation of the handheld personal care device with respect to the user during use of the handheld personal care device based on a predetermined relationship between the location and/or orientation of the handheld personal care device and the posture of the part of the body of the user.
